# EUROPEAN PATENT SPECIFICATION

(11) **EP 2 838 500 B1**
(45) Date of publication and mention of the grant of the patent: **22.05.2019**
(21) Application number: 13719673.9
(22) Date of filing: 18.04.2013
(51) Int. Cl.: A61K 8/44, A61Q 19/02, A61K 8/86

(54) **COSMETIC COMPOSITIONS COMPRISING AN ALKYLENE OXIDE DERIVATIVE AND A N-ACYL AMINO ACID COMPOUND**
KOSMETISCHE ZUSAMMENSETZUNGEN MIT EINEM ALKYLENOXIDDERIVAT UND EINER N-ACYL-AMINOSÄUREVERBINDUNG
COMPOSITIONS COSMÉTIQUES COMPRENANT UN DÉRIVÉ D'OXYDE D'ALKYLÈNE ET UN COMPOSÉ D'ACIDE N-ACYL AMINÉ

(30) Priority: 19.04.2012 US 201261635334 P
(43) Date of publication of application: 25.02.2015
(73) Proprietor: The Procter & Gamble Company, Cincinnati, OH 45202 (US)
(72) Inventor: TANAKA, Shuhei, Singapore 138648 (SG)
(74) Representative: Engisch, Gautier
(86) International application number: PCT/US2013/037075
(87) International publication number: WO 2013/158824

(56) References cited:
- WO-A2-2013/190096
- FR-A1- 2 948 872
- JP-A- 2011 126 809
- US-A1- 2010 028 471

## Description

### FIELD OF THE INVENTION

The present invention relates to cosmetic compositions containing an N-acyl amino acid compound, and more particularly related to skin irritation mitigating cosmetic composition containing an N-acyl amino acid compound and an alkylene oxide derivative, and to a compositions preparation methods thereof. Such compositions are useful for regulating the condition of mammalian keratinous tissue needing such treatments.

### BACKGROUND OF THE INVENTION

Mammalian keratinous tissue, particularly human skin, is subjected to a variety of insults by both extrinsic and intrinsic factors. Such extrinsic factors include ultraviolet radiation, environmental pollution, wind, heat, infrared radiation, low humidity, harsh surfactants, abrasives, etc. Intrinsic factors, on the other hand, include chronological aging and other biochemical changes from within the skin. Whether extrinsic or intrinsic, these factors result in visible signs of skin damage. Typical skin damage in aging or damaged skin include fine lines, wrinkling, hyperpigmentation, sallowness, sagging, dark under-eye circles, puffy eyes, enlarged pores, diminished rate of turnover, and abnormal desquamation or exfoliation. Additional damage incurred as a result of both external and internal factors includes visible dead skin i.e., flaking, scaling, dryness, and roughness.

Currently, there are a number of personal care products that are available to consumers, which are directed toward improving the health and physical appearance of keratinous tissues such as the skin, hair, and nails. The majority of these products are directed to delaying, minimizing or even eliminating skin wrinkling, spots, and other histological changes typically associated with the aging of skin or environmental damage to human skin. Consumers prefer topically applied products since they are not only effective, but also safe and pleasant to use.

Some ingredients used in cosmetics are potentially irritating which is commonly perceived as skin redness, itching, swelling, etc.

An N-acyl amino acid, particularly N-acyl derivatives of phenylalanine or tyrosine, their isomers, or their salts are known for being useful in preventing or treating various ketatinization disorders. These compounds are cosmetic active agents of choice such as for whitening products. However, it has been observed that these compounds when formulated in cosmetic compositions tend to cause skin irritation.

Skin irritation can be ameliorated by lowering the amount of an active ingredient in a composition or by formulating anti-irritant or humectants such as glycerin, however, anti-irritants would randomly work, and an anti-irritant known for a certain active ingredient may or may not effectively mitigate skin irritation caused by another active ingredient. In addition, it is also reported that inclusion of humectants may reduce the active ingredient's penetration through the skin. Therefore, these approaches have a critical drawback that the efficacy from the ingredient is impaired.

US 2007/028089A discloses an alkylene oxide derivative represented by the following formula (I) having an excellent feel and moisturizing effect, while keeping moisture even after cleansing:

Z-{O(AO)ₗ(EO)ₘ-(BO)ₙH}ₐ (I)

Z represents a residue of a compound having 3 to 9 hydroxyl groups with said hydroxyl groups removed, a is from 3 to 9, AO represents an oxyalkylene group of 3 to 4 carbon atoms, EO represents oxyethylene group, 1 and m represent average addition mol numbers of said oxyalkylene group of 3 to 4 carbon atoms and said oxyethylene group, respectively, and 1≦1≦50, 1≦m≦50, the weight ratio (AO/EO) of AO to EO is from 1/5 to 5/1, AO and EO may be added in a random form or may be added in a block form. BO represents an oxyalkylene group with a number of carbon atoms of 4, and n is an average additional mol number and 0.5≦n≦5).

Japanese unexamined patent publication No. 2010-6715A discloses a cosmetic composition for whitening comprising a water-soluble whitening active component, and an alkylene oxide derivative disclosed in US 2007/028089A, and states that the alkylene oxide derivative can enhance penetration of the water-soluble whitening component into the skin, and the composition can provide excellent whitening effect to the skin.

Based on the foregoing, there is a continuing need to formulate cosmetic compositions containing an N-acyl amino acid compound that can provide stable delivery of skin actives.

US 2010/0028471 discloses cosmetic compositions.

### SUMMARY OF THE INVENTION

The present invention relates to a cosmetic composition according to claim 1, for use in mitigating irritation of the skin upon use of said composition.

### DETAILED DESCRIPTION OF THE INVENTION

While the specification concludes with the claims particularly pointing and distinctly claiming the invention, it is believed that the present invention will be better understood from the following description.

The compositions of the present invention can include, consist essentially of, or consist of, the components of the present invention as well as other ingredients described herein.

The term "ambient conditions" as used herein refers to surrounding conditions under about one atmosphere of pressure, at about 50% relative humidity, and at about 25°C unless otherwise specified.

As used herein, the "cosmetic product(s)" and "cosmetic composition(s)" are those used to treat or care for, or somehow moisturize, improve, or clean the skin. The "cosmetic product(s)" and "cosmetic composition(s)" include, but are not limited to, moisturizers, personal cleansing products, makeup bases, foundations, occlusive drug delivery patches, nail polish, powders, wipes, hair conditioners, skin treatment emulsions, shaving creams and the like.

The term "keratinous tissue" as used herein, refers to keratin-containing layers disposed as the outermost protective covering of mammals (e.g., humans, dogs, cats, etc.) which includes, but is not limited to, skin, lips, hair, toenails, fingernails, cuticles, hooves, etc.

The term "regulating skin condition" as used herein, refers to improving skin appearance and/or feel, for example, by providing a benefit, such as a smoother appearance and/or feel. Herein, "improving skin condition" means effecting a visually and/or tactilely perceptible positive change in skin appearance and feel. The benefit may be a chronic benefit and may include one or more of the following: Reducing the appearance of wrinkles and coarse deep lines, fine lines, crevices, bumps, and large pores; thickening of keratinous tissue (e.g., building the epidermis and/or dermis and/or sub-dermal layers of the skin, and where applicable the keratinous layers of the nail and hair shaft, to reduce skin, hair, or nail atrophy); increasing the convolution of the dermal-epidermal border (also known as the rete ridges); preventing loss of skin or hair elasticity, for example, due to loss, damage and/or inactivation of functional skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by hyperpigmentation, etc.

The term "safe and effective amount" as used herein, refers to an amount of a compound or composition sufficient to significantly induce a positive benefit, preferably a positive keratinous tissue appearance or feel benefit, or positive hair appearance or feel benefit, including independently or in combinations the benefits disclosed herein, but low enough to avoid serious side effects, i.e., to provide a reasonable benefit to risk ratio, within the scope of sound judgment of the skilled artisan.

All percentages, parts and ratios are based upon the total weight of the compositions in the present invention and all measurements made are at 25°C, unless otherwise designated. All such weights as they pertain to listed ingredients are based on the active level and, therefore, do not include carriers or by-products that may be included in commercially available materials, unless otherwise specified.

The compositions in the present invention are useful for regulating the skin condition, and especially for regulating keratinous tissue condition.

The compositions in⁻ the present invention provide additional benefits, including stability, absence of significant (consumer-unacceptable) skin irritation and good aesthetics.

The compositions in the present invention optionally comprise an emulsifier, a skin active, a skin conditioning agent, a sunscreen agent, a thickening agent, and/or a non-emulsifying crosslinked siloxane elastomer.

The compositions in the present invention optionally comprise additional oil which can solubilize an N-acyl amino acid compound.

The compositions in the present invention are described in detail hereinafter.

### ALKYLENE OXIDE DERIVATIVES

The compositions of the present invention comprise a safe and effective amount of an alkylene oxide derivative represented by the following formula (I):

Z-{O(AO)ₗ(EO)ₘ-(BO)ₙH}ₐ (I)

Z represents a residue of a compound having 3 to 9 hydroxyl groups with said hydroxyl groups removed, a is from 3 to 9, AO represents an oxyalkylene group of 3 to 4 carbon atoms, EO represents oxyethylene group, 1 and m represent average addition mol numbers of said oxyalkylene group of 3 to 4 carbon atoms and said oxyethylene group, respectively, and 1≦1≦50, 1≦m≦50, the weight ratio (AO/EO) of AO to EO is from 1/5 to 5/1, AO and EO may be added in a random form or may be added in a block form. BO represents an oxyalkylene group with a number of carbon atoms of 4, and n is an average additional mol number and 0.5≦n≦5).

As used herein, one of alkylene oxide derivatives represented by formula (I), PEG/PPG/POLYBUTYLENE GLYCOL-8/5/3 Glycerin is commercially available under the tradename Wilbride S-753 from NOF Corporation.

The compositions according to the present invention comprise the alkylene oxide derivative represented by formula (I) from about 0.1% to about 20%, preferably from about 0.25% to about 10%, and more preferably from about 0.5% to about 5% by weight of the composition. Too high level of alkylene oxide derivative represented by formula (I) may bring heavy skin feel and stickiness.

### N-ACYL AMINO ACID COMPOUNDS

The compositions of the present invention comprise a safe and effective amount of one or more N-acyl amino acid compounds. The amino acid can be one of any of the amino acids known in the art. The N-acyl amino acid compounds of the present invention correspond to the formula: 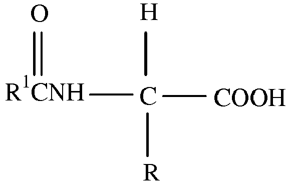 wherein R is a hydrogen, alkyl (substituted or unsubstituted, branched or straight chain), or a combination of alkyl and aromatic groups. A list of possible side chains of amino acids known in the art are described in Stryer, Biochemistry, 1981, published by W.H. Freeman and Company. R¹ is C₁ to C₃₀ , saturated or unsaturated, straight or branched, substituted or unsubstituted alkyls; substituted or unsubstituted aromatic groups; or mixtures thereof.

Preferably, the N-acyl amino acid compound is selected from the group consisting of N-acyl Phenylalanine, N-acyl Tyrosine, their isomers, their salts, derivatives thereof, and mixtures thereof. The amino acid can be the D or L isomer or a mixture thereof. N-acyl Phenylalanine corresponds to the following formula: 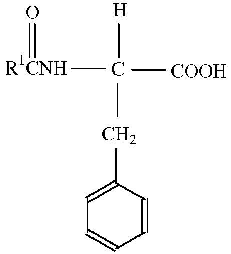 wherein R¹ is C₁ to C₃₀ , saturated or unsaturated, straight or branched, substituted or unsubstituted alkyls; substituted or unsubstituted aromatic groups; or mixtures thereof.

N-acyl Tyrosine corresponds to the following formula: 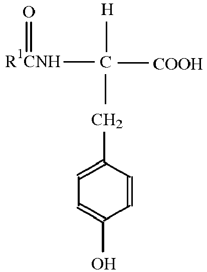 wherein R¹ is C₁ to C₃₀ , saturated or unsaturated, straight or branched, substituted or unsubstituted alkyls; substituted or unsubstituted aromatic groups; or mixtures thereof.

Particularly useful as a topical skin tone evening (lightening or pigmentation reduction) cosmetic agent is N-undecylenoyl-L-phenylalanine. This agent belongs to the broad class of N-acyl Phenylalanine derivatives, with its acyl group being a C11 mono-unsaturated fatty acid moiety and the amino acid being the L-isomer of phenylalanine. N-undecylenoyl-L-phenylalanine corresponds to the following formula:

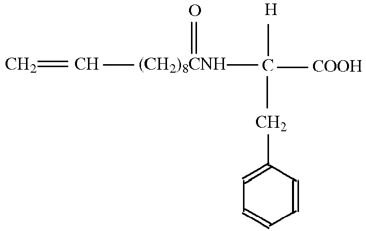

As used herein, N-undecylenoyl-L-phenylalanine is commercially available under the tradename Sepiwhite® from SEPPIC.

The compositions in the present invention comprise the N-acyl amino acid from about 0.001% to about 10%, preferably from about 0.01% to about 5%, and more preferably from about 0.1% to about 4% by weight of the composition.

### DERMATOLOGICALLY ACCEPTABLE CARRIER

The compositions in the present invention also comprise a dermatologically acceptable carrier. The dermatologically acceptable carrier may be present in an amount of from about 70% to about 99.899%, preferably from about 80% to about 85%, more preferably from about 90% to about 98.5% by weight of the composition.

The carrier can be in a wide variety of forms. For example, emulsion carriers, including, but not limited to, oil-in-water, water-in-oil, water-in-oil-in-water, oil-in-water-in-oil emulsions, aqueous solution, water gel, oil dispersion and oil gel are useful herein. Oil in the carrier herein is understood to include silicone oil.

### OPTIONAL COMPONENTS

### Skin Actives

The compositions in the present invention may include at least one skin active compound. Without being bound by theory, it is believed the present compositions provide versatility in formulating a variety of actives.

In any embodiment of the present invention, however, the actives useful herein can be categorized by the benefit they provide or by their postulated mode of action. However, it is to be understood that the actives useful herein can in some instances provide more than one benefit or operate via more than one mode of action. Therefore, classifications herein are made for the sake of convenience and are not intended to limit the active to that particular application or applications listed.

### Vitamin B₃ compounds

Vitamin B₃ compounds such as niacinamide are a preferred skin care active for use herein. The present invention preferably includes from about 0.1% to about 30%, more preferably from about 1% to about 20%, even more preferably from about 2% to about 10% of a vitamin B₃ compound.

As used herein, "vitamin B₃ compound" means a compound having the formula: 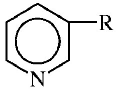 wherein R is - CONH₂ (i.e., niacinamide), - COOH (i.e., nicotinic acid) or - CH₂OH (i.e., nicotinyl alcohol); derivatives thereof; and salts of any of the foregoing. Exemplary derivatives of the foregoing vitamin B₃ compounds include nicotinic acid esters, including non-vasodilating esters of nicotinic acid (e.g., tocopheryl nicotinate), nicotinyl amino acids, nicotinyl alcohol esters of carboxylic acids, nicotinic acid N-oxide and niacinamide N-oxide.

### Whitening Agents

The present compositions may contain a whitening agent. The whitening agent useful herein refers to active ingredients that not only alter the appearance of the skin, but further improve hyperpigmentation as compared to pre-treatment. Useful whitening agents useful herein include ascorbic acid compounds, vitamin B₃ compounds, azelaic acid, butyl hydroxy anisole, gallic acid and its derivatives, hydroquinoine, kojic acid, arbutin, mulberry extract, tetrahydrocurcumin, and mixtures thereof. Use of combinations of whitening agents is also believed to be advantageous in that they may provide whitening benefit through different mechanisms.

When used, the compositions preferably contain from about 0.1% to about 10%, more preferably from about 0.2% to about 5%, by weight of the composition, of a whitening agent.

Ascorbic acid compounds are useful whitening agents, and include compounds having the formula (I): 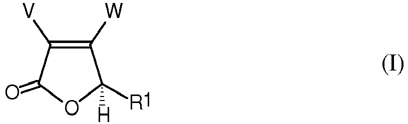 wherein V and W are independently -H or -OH; R¹ is - CH(OH)-CH₂OH; salts thereof; and derivatives thereof. Preferably, the ascorbic acid compound useful herein is an ascorbic acid salt or derivative thereof, such as the non-toxic alkali metal, alkaline earth metal and ammonium salts commonly known by those skilled in the art including, but not limited to, the sodium, potassium, lithium, calcium, magnesium, barium, ammonium and protamine salts which are prepared by methods well known in the art. Ascorbyl glucoside is a preferably derivative.

### Peptides

Peptides, including but not limited to, di-, tri-, tetra-, and pentapeptides and derivatives thereof, may be included in the compositions of the present invention in amounts that are safe and effective. As used herein, "peptides" refers to both the naturally occurring peptides and synthesized peptides. Also useful herein are naturally occurring and commercially available compositions that contain peptides.

When included in the present compositions, peptides are preferably included in amounts of from about 1x10⁻⁶% to about 10%, more preferably from about 1x10⁻⁶% to about 0.1%, even more preferably from about 1x10⁻⁵% to about 0.01 %, by weight of the composition.

### Sugar Amines

The compositions in the present invention may include a safe and effective amount of a sugar amine, which are also known as amino sugars. As used herein, "sugar amine" refers to an amine derivative of a six-carbon sugar. Examples of sugar amines that are useful herein include glucosamine, N-acetyl glucosamine, mannosamine, N-acetyl mannosamine, galactosamine, N-acetyl galactosamine. Preferred for use herein is glucosamine. Additionally, combinations of two or more sugar amines may be used.

When included in the present compositions, a sugar amine is preferably included in amounts of from about 0.001% to about 20%, more preferably from about 1% to about 10%, even more preferably from about 2% to about 5%, by weight of the composition.

### Skin Conditioning Agent

Optionally, the composition in the present invention can further comprise a skin conditioning agent. These agents may be selected from humectants and emollients. The amount of skin-condition agent may range from about 1% to about 50%, preferably from about 2% to about 40%, more preferably from about 5% to about 30%, by weight of the composition.

Humectants are polyhydric alcohols intended for moisturizing, reducing scaling and stimulating removal of built-up scale from the skin. Typical polyhydric alcohols include polyalkylene glycols and more preferably alkylene polyols and their derivatives. Illustrative are propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin and mixtures thereof. Most preferably the humectant is glycerin.

When the conditioning agent is an emollient it may be selected from hydrocarbons, fatty acids, fatty alcohols and esters.

### Sunscreen Agents

The compositions in the present invention may optionally contain a sunscreen agent selected from an organic sunscreen agent and an inorganic sunscreen agent.

Organic sunscreen agents useful herein include homosalate, octocrylene, octyl-p-methoxycinnamate, phenyl benzimidazole sulfonic acid, 2-hydroxy-4-methoxybenzophenone (Benzophenone-3), 2-ethylhexyl-salicylate, and mixtures thereof.

Inorganic sunscreen agents useful herein include the following metallic oxides; titanium dioxide, zinc oxide, zirconium oxide, iron oxide, and mixtures thereof.

When included in the present compositions, the sunscreens are preferably included in amounts of from about 0.1% to about 20%, preferably from about 0.5% to about 10%, more preferably from about 1% to about 5%, by weight of the composition. Exact amounts will vary depending upon the sunscreen or sunscreens chosen and the desired Sun Protection Factor (SPF).

### α-Monoalkyl glyceryl ether

The compositions in the present invention may optionally contain α-monoalkyl glyceryl ether to mitigate skin irritation more effectively. The α-monoalkyl glyceryl ether used in the present invention can be either straight or branched chain alkyl group having a carbon number 8-24, including batyl alcohol and chimyl alcohol. Most preferably, the α-monoalkyl glyceryl ether is batyl alcohol. When included in the present compositions, α-monoalkyl glyceryl ether is preferably included in amounts of from about 0.01% to about 10%, preferably from about 0.1% to about 7%, more preferably from about 0.2% to about 5%, by weight of the composition.

### Thickening Agents

The compositions in the present invention, in some embodiments, may further include one or more thickening agents.

Nonlimiting classes of thickening agents include those selected from the following: carboxylic acid polymers, crosslinked polyacrylate polymers, polyacrylamide polymers, polysaccharides and gums.

When present, the composition preferably includes from about 0.01% to about 5%, more preferably from about 0.1% to about 4%, and still more preferably from about 0.1% to about 3%, by weight of the composition of the thickening agent.

### Emulsifiers

The compositionin the present invention may contain an emulsifier, useful for dispersing and suspending a discontinuous phases in a continuous phase when a product is in an emulsion type. An emulsifier in the present invention can be selected from the group consisting of nonionic emulsifiers, anionic emulsifiers, cationic emulsifiers, amphoteric emulsifiers, and mixtures thereof. When the composition of the present invention contains an emulsifier, in a preferred embodiment, the composition contains from about 0.1% to about 10% emulsifier, more preferably from about 0.2% to about 7.5%, even more preferably from about 0.5% to about 5%, emulsifier by weight of the composition. A wide variety of emulsifying agents can be employed herein.

### Non-Emulsifying Crosslinked Siloxane Elastomers

The compositions in the present invention may optionally contain non-emulsifying crosslinked siloxane elastomers which have known to provide silky and smooth feeling. The term "non-emulsifying crosslinked siloxane elastomers," as used herein, defines crosslinked organopolysiloxane elastomer from which polyoxyalkylene units or polyglycerin units are absent.

Non-limiting examples of non-emulsifying crosslinked siloxane elastomers used herein include dimethicone/vinyl dimethicone crosspolymers, supplied by a variety of suppliers including Dow Corning™ (DC 9040 and DC 9041), General Electric™ (SFE 839), Shin-Etsu™ (KSG-15, 16, 18 [dimethicone/phenyl vinyl dimethicone crosspolymer]), and Grant Industries (GRANSIL™ line of elastomers). Cross-linked siloxane elastomers useful in the present invention and processes for making them are further described in US Patent 4,970,252 to Sakuta, et al.; US Patent 5,760,116 to Kilgour, et al.; and US Patent 5,654,362 to Schulz, Jr., et al. issued August 5, 1997. Additional crosslinked organopolysiloxane elastomers useful in the present invention are disclosed in Japanese Patent Application JP 61-18708, assigned to Pola Kasei Kogyo KK.

In some embodiments, the composition may contain from about 0.1% to about 15%, preferably from about 0.1% to about 10%, most preferably from about 1% to about 6% of a non-emulsifying crosslinked siloxane elastomer by weight of the composition.

### Other Optional Ingredients

A variety of additional ingredients can be incorporated into the compositions in the present invention. Nonlimiting examples of these additional ingredients include; particular materials to modify skin feel or appearance; anti-acne actives; oil-soluble beta-hydroxy acids such as salicylic acid and derivatives thereof; chelators; flavonoid compounds; anti-inflammatory agents; anti-cellulite agents; desquamation actives; anti-oxidant/radical scavengers; tanning actives; skin soothing or skin healing actives such as panthenoic acid derivatives (including panthenol, dexpanthenol, ethyl panthenol), aloe vera, allantoin, bisabolol, and dipotassium glycyrrhizinate; antimicrobial or antifungal actives.

### COMPOSITION PREPARATION

The compositions in the present invention are generally prepared by conventional methods such as are known in the art of making topical compositions. Such methods typically involve mixing of the ingredients in one or more steps to a relatively uniform state, with or without heating, cooling, application of vacuum, and the like.

### PRODUCTS FOR TOPICAL USE

The topical compositions in the present invention may be formulated into cosmetic products such as foundations, moisturizer, wrinkle soothing serum, lotion, skin facial mask, skin lotion, skin cream, skin gel, eye gel, eye cream, or any other commonly known skin product or treatment.

In some embodiments, the compositions in the present invention are in the form of water-in-oil emulsion wherein an oil phase comprises an N-acyl amino acid compound and a water phase comprises an alkylene oxide derivative represented by formula (I).

In some embodiments, the compositions in the present invention are in the form of oil-in-water emulsion wherein a water phase comprises an N-acyl amino acid compound and an alkylene oxide derivative represented by formula (I).

In some embodiments, the compositions in the present invention are in the form of oil-in-water emulsion wherein an oil phase comprises an N-acyl amino acid compound and an alkylene oxide derivative represented by formula (I).

In some embodiments, the compositions in the present invention further comprises batyl alcohol, preferably in the range of from about 0.01% to about 10%.

### TEST METHOD

### Closed Patch Test

### Procedure

2 cm x 2 cm nonwoven cotton pads (e.g. Webril by Covidien) are prepared to include 0.2 g of test substance in each patch. A total of 5 patches are applied to each arm. Patches containing test substances are applied to the lateral surface of the upper arms. The site of application is marked in such a way that the location of the patch pad is clearly indicated for scoring. To assure good adhesion, patches may be reinforced with a hypoallergenic tape (e.g. Dermacil®, Micropore®, etc.). If extra tape is used, it should be applied only over existing tape, not directly over the patch pad. Patches remains 24 hours on the skin, and should be kept dry while being worn. Upon removing the patches 24 hours after application, skin sites are rinsed with water to remove excess test material and patted dry. Skin sites are graded 30 minutes following patch removal.

### Grading of the Test Sites

A lamp with an incandescent, 60 watt daylight bulb, is used to illuminate the skin where a patch was applied. Any visible response is palpated to aid in detecting signs of elevation. The scoring scale to be used is shown below. The same trained grader scores all sites throughout the test.
0 No apparent cutaneous involvement.
0.5 Faint, barely perceptible erythema or slight dryness (glazed appearance).
1 Faint but definite erythema, no eruptions or broken skin or no erythema but definite dryness; may have epidermal fissuring.
1.5 Well-defined erythema or faint erythema with definite dryness, may have epidermal fissuring.
2 Moderate erythema, may have a few papules or deep fissures, moderate-to-severe erythema in the cracks.
2.5 Moderate erythema with barely perceptible edema or severe erythema not involving a significant portion of the patch (halo effect around the edges), may have a few papules or moderate-to-severe erythema.
3 Severe erythema (beet redness), may have generalized papules or moderate-to-severe erythema with slight edema (edges well defined by raising).
3.5 Moderate-to-severe erythema with moderate edema (confined to patch area) or moderate-to-severe erythema with isolated eschar formations or vesicles.
4 Generalized vesicles or eschar formations or moderate-to-severe erythema and/or edema extending beyond the area of the patch.
Average score for each test substance is obtained by adding scores from patches containing the same test substance, and dividing the obtained score by a total number of a tested patch containing the same substance.

### In vitro Skin Active Penetration

In-vitro Frenz cell test is used to measure the amount of penetration of the radio labeled N-undecylenoyl-L-phenylalanine through the cadaver skin. The frozen human cadaver skin from the selected donor is thawed at ambient conditions, and rinsed with copious distilled water to remove glycerin preservative. The receptors [∼ 5 mL] will be filled with 80/20 water/ethanol and the skin allowed to equilibrate for two hours. Approximately 5 µL of each test product is applied to the individual cells. The receptor solution is collected and replaced at 2 and 4 hours or 2, 4, and 6 hours, with a final collection at 24 hours. At the end of the study (24 hr), each skin sample is wiped three times with Whatman filter paper soaked with 80%/20% ethanol water to remove unabsorbed (residual) product. The epidermis subsequently is dissected from the residual dermis. All skin sections and filter paper wipes is extracted twice with 5 mL ethanol (∼24 hr and ∼72 hr extraction times). All receptor solution collections and ethanol extracts is assayed using a HPLC/MS/MS based assay.

### EXAMPLES

### Compositions

The following examples further describe and demonstrate embodiments within the scope of the present invention. The examples are given solely for the purpose of illustration and are not to be construed as limitations of the present invention, as many variations thereof are possible without departing from the spirit and scope of the invention

Compositions were prepared by conventional methods from the following components.

| | Ex 1 | Ex 2 | Ex3 | EX 4 | Ex5 | Ex6 | Com. Ex 1 |
|---|---|---|---|---|---|---|---|
| Water Purified | 59.98 | 59.61 | 58.86 | 58.86 | 58.26 | 58.31 | 54.36 |
| Niacinamide | 5.0000 | 5.0000 | 5.0000 | 5.0000 | 5.0000 | 5.0000 | 5.0000 |
| Hexyldecanol | 5.0000 | 5.0000 | 5.0000 | 5.0000 | 5.0000 | 5.0000 | 5.0000 |
| Glycerin | 7.0000 | 7.0000 | 7.0000 | 7.0000 | 7.0000 | 7.0000 | 7.0000 |
| KSG-15 ^{*1} | 3.5000 | 3.5000 | 3.5000 | 3.5000 | 3.5000 | 3.5000 | 3.5000 |
| Pentylene Glycol | 3.0000 | 3.0000 | 3.0000 | 3.0000 | 3.0000 | 3.0000 | 3.0000 |
| 1,3-Butylene Glycol | 2.0000 | 2.0000 | 2.0000 | 2.0000 | 2.0000 | 2.0000 | 2.0000 |
| Isopropyl Isostearate | 2.0000 | 2.0000 | 2.0000 | 2.0000 | 2.0000 | 2.0000 | 2.0000 |
| Inositol | 0.3000 | 0.3000 | 0.3000 | 0.3000 | 0.3000 | 0.3000 | 0.3000 |
| Nylon powder SP-500^{*2} | 1.5000 | 1.5000 | 1.5000 | 1.5000 | 1.5000 | 1.5000 | 1.5000 |
| WILBRIDE S-753^{*3} | 1.5000 | 0.7500 | 2.0000 | 2.0000 | 2.0000 | 2.0000 | 0.0000 |
| White Petrolatum | 1.5000 | 1.5000 | 1.5000 | 1.5000 | 1.5000 | 1.5000 | 0.0000 |
| Eldew PS-203^{*4} | 1.0000 | 1.0000 | 1.0000 | 1.0000 | 1.0000 | 1.0000 | 1.0000 |
| Sepigel 305 ^{*5} | 0.6000 | 0.6000 | 0.6000 | 0.6000 | 0.6000 | 0.6000 | 0.6000 |
| Tocopheryl Acetate | 0.5000 | 0.5000 | 0.5000 | 0.5000 | 0.5000 | 0.5000 | 0.5000 |
| Polysorbate 20 | 1.0000 | 1.0000 | 1.0000 | 1.0000 | 1.0000 | 1.0000 | 1.0000 |
| Dexpanthenol | 1.0000 | 1.0000 | 1.0000 | 1.0000 | 1.0000 | 1.0000 | 1.0000 |
| Ultrez 20 ^{*6} | 0.2000 | 0.2000 | 0.2000 | 0.2000 | 0.2000 | 0.2000 | 0.2000 |
| Benzyl Alcohol | 0.2000 | 0.2000 | 0.2000 | 0.2000 | 0.2000 | 0.2000 | 0.2000 |
| Pemulen TR-2 ^{*7} | 0.2000 | 0.2000 | 0.2000 | 0.2000 | 0.2000 | 0.2000 | 0.2000 |
| Sepiwhite MSH ^{*8} | 0.2000 | 1.0000 | 1.0000 | 1.0000 | 1.0000 | 1.0000 | 1.0000 |
| Hexylene Glycol | 0.7500 | 0.7500 | 0.7500 | 0.7500 | 0.7500 | 0.7500 | 0.7500 |
| Hexandiol | 0.2500 | 0.2500 | 0.2500 | 0.2500 | 0.2500 | 0.2500 | |
| Lipidure PMB(ph10) ^{*9} | 0.5000 | 0.5000 | 0.5000 | 0.0000 | 0.5000 | 0.5000 | 0.0000 |
| Aminomethylpropanol | 0.2300 | 0.5500 | 0.5500 | 0.5500 | 0.5500 | 0.5500 | 0.5500 |
| EDTA-2NA | 0.1000 | 0.1000 | 0.1000 | 0.1000 | 0.1000 | 0.1000 | |
| Phenoxyethanol | 0.2500 | 0.2500 | 0.2500 | 0.2500 | 0.2500 | 0.2500 | 0.2500 |
| Xanthan gum | 0.0500 | 0.0500 | 0.0500 | 0.0500 | 0.0500 | 0.0500 | 0.0500 |
| Sodium Benzoate | 0.0500 | 0.0500 | 0.0500 | 0.0500 | 0.0500 | 0.0500 | 0.0500 |
| Allantoin | | | | | 0.1000 | | |
| Glycyrretinic Acid | | | | | | 0.0500 | |
| Batyl Alcohol | | | | | | | |
| Ascorbyl Glucoside | 0.0250 | 0.0250 | 0.0250 | 0.0250 | 0.0250 | 0.0250 | 0.0250 |
| Perfume | 0.1150 | 0.1150 | 0.1150 | 0.1150 | 0.1150 | 0.1150 | 0.1150 |

| | | | | | | | |
|---|---|---|---|---|---|---|---|
| *1 KSG-15 (Cyclopentasiloxane and Dimethicone/Vinyl Dimethicone Crosspolymer,): Available from Shin-Etsu Chemical Co., *2 Nylon powder SP-500 (Nylon-12): Mizushima Goukin *3 WILBRIDE S-753 (PEG/PPG/POLYBUTYLENE GLYCOL-8/5/3 GLYCERIN): Available from NOF Co. *4 Eldew PS-203 (Phytosteryl/Octyldodecyl Lauroyl Glutamate (and) Tocopherol): Available from Ajinomoto *5 Sepigel 305 (Polyacrylamide/C13-14 Isoparaffin/Laureth-7): Available from SEPPIC Inc. *6 Ultrez 20 (Acrylates/C10-30 Alkyl Acrylate Crosspolymer): Available from Noveon *7 Pemulen TR-2 (Acrylates/C10-30 Alkyl Acrylate Crosspolymer): Available from Noveon Inc. *8 Sepiwhite MSH (Undecylenoyl Phenyl Alanine): Available from Seppic. *9 Lipidure PMB(ph10) (2-(Methacryloyloxyethyl)-2'-(trimethyl-ammoniumethyl) phosphate, inner salt -n-butylmethacrylate copolymer): Available from NOF Co. | | | | | | | |

Compositions of examples 1-6 and comparative example 1 are suitably made as follows:
(1) Phase A (Water Phase): Water, Niacinamide, Glycerin, 1,3-Butylene Glycol, Inositol, WILBRIDE S-753, Polysorbate 20, Dexpanthenol, Ultrez 20, Benzyl alcohol, Pemulen TR-2, Sepiwhite MSH, Sepigel 305, Hexylene Glycol, 1,2 Hexane diol, Lipidure PMB (pH), Aminometylpropanol, EDTA-2Na, Phenoxyethanol, Xanthan Gum, Sodium Benzoate, Allantoin and Ascorbyl Glucoside were mixed in a separate vessel using a suitable mixer (e.g., Anchor blade, propeller blade or IKA T25) until the phase was homogenous.
(2) Phase B (Oil Phase): Hexyldecanol, Isopropyl Isostearate, White Petrolatum, Tocopheryl Acetate, Eldew PS-203, Glycyrretinic Acid, Batyl Alcohol were mixed in a vessel using a suitable mixer (e.g., Anchor blade, propeller blade or IKA T25) by heating at 70C until the phase was homogenous.
(3) Phase C (Powder phase): Nylon powder SP-500 and pentylene glycol were mixed in a separate vessel until the phase was homogenous.
(4) Phase B was added slowly into Phase A, and the obtained mixture was mixed until the batch became homogenous. Then phase C and Perfume were added into the mixture of Phase A and Phase B and mixed until the batch became homogenous.

### Closed Patch Test I

According to Closed Patch Test described above, with 32 subjects, skin irritation of compositions of examples 1, 2 and comparative example 1 were measured.

| | Ex 1 | Ex 2 | Ex 3 | Com. Ex 1 |
|---|---|---|---|---|
| Skin irritation score | 0.33 | 1.16 | 0.95 | 1.38 |

### Closed Patch Test II

According to Closed Patch Test described above, with 33 subjects, another test to measure skin irritation of compositions of examples 3-7 were measured.

| | Ex3 | Ex4 | Ex 5 | Ex 6 |
|---|---|---|---|---|
| Skin irritation score | 1.32 | 1.02 | 1.35 | 1.15 |

### Skin Active Penetration

According to In vitro Skin Active Penetration described above, N-undecylenoyl-L-phenylalanine penetration using compositions of example 1 and comparison example 3 were measured using 6 different human cadaver skin samples.

| N-undecylenoyl-L-phenylalanine penetration (ug/cm²) of total skin at 24 hours | Ex 3 | Com. Ex 1 |
|---|---|---|
| Sample 1 | 8.53 | 10.82 |
| Sample 2 | 8.07 | 8.36 |
| Sample 3 | 23.28 | 6.65 |
| Sample 4 | 26.74 | 32.08 |
| Sample 5 | 35.60 | 35.41 |
| Sample 6 | 39.16 | 34.75 |
| Average | 21.34 | 23.56 |
| p value | | 0.67 |

N-undecylenoyl-L-phenylalanine penetration of the compositions of example 3 and comparative example 1 are not statistically different.
dimension is intended to mean both the recited value and a functionally equivalent range surrounding that value. For example, a dimension disclosed as "40 mm" is intended to mean "about 40 mm." The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A composition comprising
a) from 0.1% to 20% of an alkylene oxide derivative represented by the following formula (I):
Z-{O(AO)₁(EO)ₘ-(BO)ₙH}ₐ (I)
Wherein Z represents a residue of a compound having 3 to 9 hydroxyl groups with said hydroxyl groups removed, a is from 3 to 9, AO represents an oxyalkylene group of 3 to 4 carbon atoms, EO represents oxyethylene group, 1 and m represent average addition mol numbers of said oxyalkylene group of 3 to 4 carbon atoms and said oxyethylene group, respectively, and 1≦1≦50, l≦m≦50, the weight ratio (AO/EO) of AO to EO is from 1/5 to 5/1, AO and EO may be added in a random form or may be added in a block form, BO represents an oxyalkylene group with a number of carbon atoms of 4, and n is an average additional mol number and 0.5≦n≦5, in a cosmetic composition comprising
b) from 0.001% to 10% of an N-acyl amino acid compound according to the formula 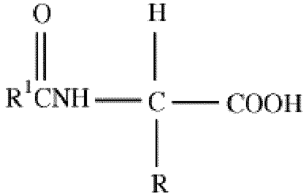 wherein R is a hydrogen, alkyl (substituted or unsubstituted, branched or straight chain), or a combination of alkyl and aromatic groups; and R¹ is C₁ to C₃₀, saturated or unsaturated, straight or branched, substituted or unsubstituted alkyls; substituted or unsubstituted aromatic groups; or mixtures thereof; and
c) dermatologically acceptable carrier, for use in mitigating irritation of the skin upon use of said composition.

2. Composition according to claim 1, wherein AO and EO in said alkylene oxide derivatives are added in a random form.

3. Composition according to claim 1, wherein AO in said alkylene oxide derivatives is oxypropylene.

4. Composition according to claim 1, wherein said alkylene oxide derivative is in the range of from 0.5% to 15%.

5. Composition according to claim 1, wherein said N-acyl amino acid is selected from the group consisting of N-acyl phenylalanine, N-acyl tyrosine, their isomers, their salts, derivatives thereof, and mixtures thereof.

6. Composition according to claim 5, wherein said N-acyl phenylalanine is N- undecylenoyl-L-phenylalanine.

7. Composition according to claim 1, wherein said composition comprises said N-acyl amino acid compound in the range of from 0.01 % to 5% by weight of the composition.

8. Composition according to claim 1, wherein said composition further comprises a skin care active selected from the group consisting of vitamin B₃ compounds, whitening agents, peptides, sugar amines, and mixtures thereof.

9. Composition according to claim 8, wherein said skin care active is a vitamin B₃ compound.

10. Composition according to claim 9, wherein said vitamin B₃ compound is a niacinamide.

11. Composition according to claim 1, wherein said composition further comprises a skin conditioning agent.

12. Composition according to claim 1, wherein said composition further comprises batyl alcohol.

13. Composition according to claim 1, wherein said composition is oil-in-water emulsion.

14. Composition according to claim 1, wherein said composition is water-in-oil emulsion.

## Patentansprüche

1. Kosmetikzusammensetzung, die Folgendes umfasst:
a) von 0,1 % bis 20 % ein Alkylenoxidderivat, dargestellt durch die folgende Formel (I):
Z-{O(AO)ₗ(EO)ₘ-(BO)ₙH}ₐ (I)
worin Z einen Rest einer Verbindung mit 3 bis 9 Hydroxylgruppen darstellt, wobei die Hydroxylgruppen entfernt sind, a von 3 bis 9 beträgt, AO eine Oxyalkylengruppe mit 3 bis 4 Kohlenstoffatomen darstellt, EO eine Oxyethylengruppe darstellt, l und m jeweils durchschnittliche hinzugefügte Molzahlen der Oxyalkylengruppe
mit 3 bis 4 Kohlenstoffatomen und die Oxyethylengruppe darstellen, und
1≦l≦50, 1≦m≦50, wobei das Gewichtsverhältnis (AO/EO) von AO zu EO von 1/5 bis 5/1 beträgt, AO und EO in einer beliebigen Form hinzugefügt werden können oder in einer Blockform hinzugefügt werden können, BO eine Oxyalkylengruppe mit einer Anzahl von Kohlenstoffatomen von 4 darstellt, und n eine zusätzliche Molzahl und 0,5≦n≦5) ist, in einer kosmetischen Zusammensetzung, die Folgendes umfasst:
b) von 0,001 % bis 10 % eine N-Acylaminosäureverbindung gemäß der Formel 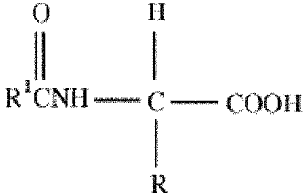 worin R ein Wasserstoff, Alkyl (substituiert oder unsubstituiert, verzweigt oder geradkettig) oder eine Kombination von Alkyl- und aromatischen Gruppen ist; und R¹ C₁- bis C₃₀-, gesättigte oder ungesättigte, gerade oder verzweigte, substituierte oder unsubstituierte Alkyle; substituierte oder unsubstituierte aromatische Gruppen; oder Mischungen davon ist; und
c) dermatologisch akzeptablen Träger, zur Verwendung für eine Minderung der Reizung der Haut bei Verwendung der Zusammensetzung.

2. Zusammensetzung nach Anspruch 1, wobei AO und EO in den Alkylenoxidderivaten in einer zufälligen Form zugegeben werden.

3. Zusammensetzung nach Anspruch 1, wobei AO in den Alkylenoxidderivaten Oxypropylen ist.

4. Zusammensetzung nach Anspruch 1, wobei das Alkylenoxidderivat in dem Bereich von 0,5 % bis 15 % beträgt.

5. Zusammensetzung nach Anspruch 1, wobei die N-Acylaminosäure ausgewählt ist aus der Gruppe bestehend aus N-Acylphenylalanin, N-Acyltyrosin, deren Isomeren, deren Salzen, Derivaten davon und Mischungen davon.

6. Zusammensetzung nach Anspruch 5, wobei das N-Acylphenylalanin N-Undecylenoyl-L-phenylalanin ist.

7. Zusammensetzung nach Anspruch 1, wobei die Zusammensetzung die N-Acylaminosäureverbindung im Bereich von 0,01 Gew. -% bis 5 Gew. -% der Zusammensetzung umfasst.

8. Zusammensetzung nach Anspruch 1, wobei die Zusammensetzung weiter einen Hautpflegewirkstoff umfasst, ausgewählt aus der Gruppe bestehend aus Vitamin-B₃-Verbindungen, Weißmachern, Peptiden, Zuckeraminen und Mischungen davon.

9. Zusammensetzung nach Anspruch 8, wobei der Hautpflegewirkstoff eine Vitamin B₃-Verbindung ist.

10. Zusammensetzung nach Anspruch 9, wobei die Vitamin B₃-Verbindung ein Niacinamid ist.

11. Zusammensetzung nach Anspruch 1, wobei die Zusammensetzung ferner ein Hautkonditioniermittel umfasst.

12. Zusammensetzung nach Anspruch 1, wobei die Zusammensetzung ferner Batylalkohol umfasst.

13. Zusammensetzung nach Anspruch 1, wobei die Zusammensetzung eine Öl-in-Wasser-Emulsion ist.

14. Zusammensetzung nach Anspruch 1, wobei die Zusammensetzung eine Wasser-in-Öl-Emulsion ist.

## Revendications

1. Composition cosmétique comprenant
a) de 0,1 % à 20 % d'un dérivé d'oxyde d'alkylène représenté par la formule (I) suivante :
Z-{O(AO)₁(EO)ₘ-(BO)ₙH}a (I)
dans laquelle Z représente un résidu d'un composé ayant 3 à 9 groupes hydroxyle avec lesdits groupes hydroxyle retirés, a va de 3 à 9, AO représente un groupe oxyalkylène de 3 à 4 atomes de carbone, EO représente un groupe oxyéthylène, l et m représentent des nombres moyens de moles d'addition dudit groupe oxyalkylène de 3 à 4 atomes de carbone et dudit groupe oxyéthylène, respectivement, et 1≦l≦50, 1≦m≦50, le rapport pondéral (AO/EO) de AO à EO va de 1/5 à 5/1, AO et EO peuvent être ajoutés sous une forme aléatoire ou peuvent être ajoutés sous une forme séquencée. BO représente un groupe oxyalkylène avec un nombre d'atomes de carbone de 4, et n est un nombre moyen de moles additionnelles et 0,5≦n≦5), dans une composition cosmétique comprenant
b) de 0,001 % à 10 % d'un composé acide N-acyl aminé selon la formule 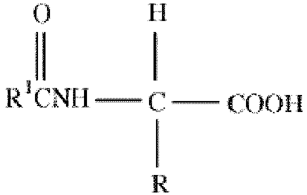 dans laquelle R est un hydrogène, un alkyle (substitué ou non substitué, à chaîne ramifiée ou linéaire), ou une combinaison de groupes alkyle et aromatiques ; et R¹ est des alkyles substitués ou non substitués, saturés ou insaturés, linéaires ou ramifiés en C₁ à C₃₀ ; des groupes aromatiques substitués ou non substitués ; ou leurs mélanges ; et
c) un véhicule acceptable du point de vue dermatologique, destiné à être utilisé pour atténuer une irritation de la peau lors d'une utilisation de ladite composition.

2. Composition selon la revendication 1, dans laquelle AO et EO dans lesdits dérivés d'oxyde d'alkylène sont ajoutés sous une forme aléatoire.

3. Composition selon la revendication 1, dans laquelle AO dans lesdits dérivés d'oxyde d'alkylène est oxypropylène.

4. Composition selon la revendication 1, dans laquelle ledit dérivé d'oxyde d'alkylène est dans la plage allant de 0,5 % à 15 %.

5. Composition selon la revendication 1, dans laquelle ledit acide N-acyl aminé est choisi dans le groupe constitué de N-acyl phénylalanine, N-acyl tyrosine, leurs isomères, leurs sels, des dérivés celles-ci, et des mélanges de celles-ci.

6. Composition selon la revendication 5, dans laquelle ladite N-acyl phénylalanine est la N-undécylénoyl-L-phénylalanine.

7. Composition selon la revendication 1, dans laquelle ladite composition comprend ledit composé acide N-acyl aminé dans la plage allant de 0,01 % à 5 %, en poids de la composition.

8. Composition selon la revendication 1, dans laquelle ladite composition comprend en outre un agent actif pour le soin de la peau choisi dans le groupe constitué de composés de vitamine B₃, agents de blanchissement, peptides, amines de sucre, et leurs mélanges.

9. Composition selon la revendication 8, dans laquelle ledit principe actif pour le soin de la peau est un composé de vitamine B₃.

10. Composition selon la revendication 9, dans laquelle ledit composé de vitamine B₃ est un niacinamide.

11. Composition selon la revendication 1, dans laquelle ladite composition comprend en outre un agent de conditionnement de la peau.

12. Composition selon la revendication 1, dans laquelle ladite composition comprend en outre de l'alcool batylique.

13. Composition selon la revendication 1, dans laquelle ladite composition est une émulsion huile-dans-eau.

14. Composition selon la revendication 1, dans laquelle ladite composition est une émulsion eau-dans-huile.
